# EUROPEAN PATENT APPLICATION

(11) **EP 0 613 617 A1**
(43) Date of publication of application: **07.09.1994**
(21) Application number: 94301338.3
(22) Date of filing: 24.02.1994
(51) Int. Cl.: A01M 1/24, A61L 9/14, B05B 7/24

(54) **Atmosphere treatment apparatus**

(30) Priority: 05.03.1993 GB 9304481
(71) Applicant: The BOC Group plc, Windlesham Surrey GU20 6HJ (GB)
(72) Inventor: Gabzdyl, Jacek Tadeusz, Guildford, Surrey GU3 3AZ (GB)
(74) Representative: Gough, Peter

(57) **Abstract**

An apparatus for delivering a fluid, for example, an aromatic compound into an atmosphere comprises a pressurised cylinder 4 containing said fluid dissolved in a liquefied carrier gas, a valve 12 for controlling the flow of the fluid and the liquefied carrier gas from the cylinder 4 to a spray nozzle 6 for delivery to the atmosphere and a timer device controlling the opening and closing of the valve 12; the pressurised cylinder 4, the valve 12, the timer device 14 and the spray nozzle 6 all being located within a housing 2 thereby enabling the apparatus to be self-contained and easily manoeuvrable.

## Description

The present invention relates to apparatus for delivering a fluid, for example, an aromatic compound into an atmosphere.

It is known to provide aerosols which are operated automatically on an intermittent basis for delivering chemicals into an atmosphere, for example, a lavatory for disinfectant purposes or for sweetening the atmosphere in the lavatory. Such aerosols because of their size tend to have a short life span and need to be replaced frequently.

In UK published patent application 2240042 there is described an apparatus for exterminating pests in for example, agricultural greenhouses. The apparatus includes a cylinder filled with liquefied carbon dioxide in which is dissolved a pesticide. The cylinder is disposed outdoors and a cylinder valve is mounted on the upper end thereof. The cylinder valve is coupled to a solenoid valve which is operated by a timer.

The arrangement is said to enable the pesticide to be sprayed inside an agricultural greenhouse at preselected time intervals.

The apparatus described suffers from the disadvantage that it is neither easily manoeuvrable nor self-contained.

It is an aim of the present invention to provide an apparatus which is portable and can stand alone for delivering a fluid such as an aromatic or deodorising compound to atmosphere in, for example, a supermarket.

According to the present invention an apparatus for delivering a fluid into an atmosphere comprises a pressurised cylinder containing the fluid dissolved in a liquefied carrier gas, a valve for controlling the flow of the fluid and liquefied carrier gas from the cylinder to a spray nozzle for delivery to the atmosphere, the valve being operated by a timer device, the apparatus being characterised in that the pressurised cylinder, the valve, the timer device and the spray nozzle are all located within a housing.

In a preferred embodiment, the housing is provided with wheels and a handle for ease of manoeuvrability.

Preferably, the timer device is powered by a battery also located in the housing. Alternatively, the timer device can be powered by an external source of power.

An embodiment of the invention will now be described by way of example reference being made to the Figures of the accompanying diagrammatic drawing in which:

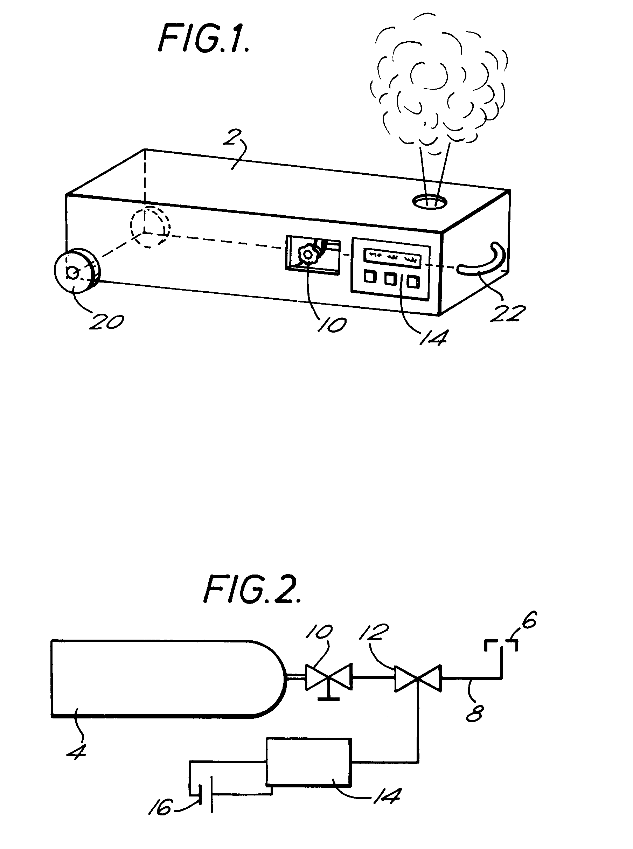

Figure 1 is a schematic view of an apparatus for delivering a fluid into the atmosphere; and
Figure 2 is a schematic view of the apparatus of Figure 1 with a housing removed.

As shown in the Figures, an apparatus for delivering, for example, an aromatic compound into the atmosphere within a supermarket comprises a housing 2 in which is located a pressurised cylinder 4 containing the aromatic compound dissolved in a liquefied carrier gas, for example, liquid carbon dioxide. Spaced from the cylinder 4 is a spray nozzle 6 which communicates with the cylinder 4 by means of line 8. Located within the line 8 is a cylinder valve 10 and a solenoid valve 12.

The solenoid valve 12 is connected to a timer 14 which in one embodiment of the invention is driven by a battery 16 also located within the housing 2.

Attached to the housing 2 are wheels 20 and a handle 22.

In use, the apparatus by means of the wheels 20 and handle 22 is positioned in a suitable location in, for example, a supermarket. Next the timer controls are operated to ensure that the aromatic compound is delivered to atmosphere for a preselected time at preselected intervals of time. Lastly, the valve 10 is moved to its open position via an aperture in the housing 2.

As already explained the timer 14 is driven in the embodiment described by a battery 16 and therefore the apparatus is capable of operating without any outside power supply.

At preselected times the timer will open the solenoid valve 12 such that a quantity of aromatic compound dissolved in liquid carbon dioxide leaves the pressurised cylinder 4 passing through valves 10, 12 and is delivered to the spray nozzle 6 for discharge to the atmosphere. The spray nozzle 6 is a fine nozzle such that the liquid carbon dioxide vaporises almost instantly creating a very fine dispersion of the aromatic compound which diffuses through the atmosphere efficiently and quickly.

When the pressurised cylinder 4 is exhausted the valve 10 is closed and a fresh cylinder 4 is provided in the housing 2. The valve 10 is then turned on, that is, moved to its open position and the apparatus is again ready for use.

It will be evident that the embodiment described above provides a portable, stand alone automatic system for introducing various chemical compounds such as aromatic compounds, deodorising compounds or disinfecting compounds into an atmosphere without the need for external power.

It will be evident that in certain circumstances the timer could be driven by, for example, mains power in which case the battery 16 need not be provided.

## Claims

1. An apparatus for delivering a fluid into an atmosphere comprising a pressurised cylinder 4 containing the fluid dissolved in a liquefied carrier gas, a valve 12 for controlling the flow of the fluid and liquefied carrier gas from the cylinder 4 to a spray nozzle 6 for delivery to the atmosphere, the valve 12 being operated by a timer device 14 characterised in that the pressurised cylinder 4, the valve 12, the timer device 14 and the spray nozzle 6 are all located within a housing 2.

2. An apparatus as claimed in Claim 1, characterised in that the housing 2 is provided with wheels 20 and a handle 22 for ease of manoeuvrability.

3. An apparatus as claimed in Claim 1 or 2, characterised in that the timer device 14 is powered by a battery 16.

4. An apparatus as claimed in Claim 1 or 2, characterised in that the timer device 14 is powered by an external source of power.

5. An apparatus as claimed in any one of Claims 1 to 4, characterised in that the liquefied carrier gas is liquefied carbon dioxide.
